(19) Europäisches Patentamt
European Patent Office
Office européen des brevets

(11) **EP 2 869 753 B1**

(12) **FASCICULE DE BREVET EUROPEEN**

(45) Date de publication et mention de la délivrance du brevet:
**07.11.2018 Bulletin 2018/45**

(21) Numéro de dépôt: **13744691.0**

(22) Date de dépôt: **04.07.2013**

(51) Int Cl.:
*A61B 3/00* (2006.01)        *A61B 3/10* (2006.01)
*A61B 3/08* (2006.01)        *A61B 3/09* (2006.01)
*A61B 3/103* (2006.01)      *G02C 13/00* (2006.01)

(86) Numéro de dépôt international:
**PCT/FR2013/051596**

(87) Numéro de publication internationale:
**WO 2014/006342 (09.01.2014 Gazette 2014/02)**

(54) **DISPOSITIF ET PROCÉDÉ DE MESURE D'AU MOINS UNE CARACTÉRISTIQUE DE RÉFRACTION OCULAIRE OBJECTIVE D'UN SUJET POUR UNE PLURALITÉ DE DISTANCES DE VISION**

VORRICHTUNG UND VERFAHREN ZUM MESSEN VON MINDESTENS EINER ZIELREFRAKTIONSEIGENSCHAFT DES AUGES EINES PATIENTEN FÜR MEHRERE VISUELLE BEREICHE

DEVICE AND METHOD FOR MEASURING AT LEAST ONE OBJECTIVE OCULAR REFRACTION CHARACTERISTIC OF A PATIENT FOR A PLURALITY OF VISUAL RANGES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.07.2012 FR 1201926**

(43) Date de publication de la demande:
**13.05.2015 Bulletin 2015/20**

(73) Titulaire: **Essilor International**
**94220 Charenton-le-Pont (FR)**

(72) Inventeurs:
• **BARANTON, Konogan**
**94220 Charenton-Le-Pont (FR)**
• **ROUSSEAU, Benjamin**
**94220 Charenton-Le-Pont (FR)**
• **DIVO, Fabien**
**94220 Charenton-Le-Pont (FR)**
• **ESCALIER, Guilhem**
**94220 Charenton-Le-Pont (FR)**

(74) Mandataire: **Jacobacci Coralis Harle**
**32, rue de l'Arcade**
**75008 Paris (FR)**

(56) Documents cités:
EP-A1- 0 305 238        FR-A1- 2 914 173
FR-A1- 2 952 517        US-A1- 2003 108 350
US-A1- 2006 244 911

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

DOMAINE TECHNIQUE AUQUEL SE RAPPORTE L'INVENTION

La présente invention concerne de manière générale le domaine des appareils et procédés de mesure de réfraction oculaire d'un sujet. Elle concerne plus particulièrement un appareil et un procédé de mesure de réfraction oculaire dans un ou plusieurs comportements visuels d'un sujet, par exemple en vision de loin (VL) et en vision de près (VP), sans point d'appui de la tête du sujet. Un opticien, un ophtalmologue ou un optométriste sont les principaux utilisateurs de l'invention.

ARRIERE-PLAN TECHNOLOGIQUE

La détermination précise des paramètres de réfraction oculaire d'un individu dans différentes postures de tête et/ou pour différents comportements visuels est essentielle dans la fabrication de lunettes de correction visuelle adaptées à ces différentes postures et/ou comportements visuels.

On connaît différents appareils et procédés de mesure de la réfraction oculaire qui permettent une mesure de la réfraction oculaire objective d'un porteur pour différents comportements visuels et/ou postures de tête. Ces appareils sont basés sur différentes techniques, par exemple la photoréfraction, ou sur la technique de skiascopie. En particulier, il est connu d'utiliser un autoréfractomètre, un aberromètre, un skiascope ou un appareil de photoréfraction afin de mesurer la réfraction objective des yeux d'un individu dans au moins un comportement visuel.

Un autoréfractomètre peut permettre dans des conditions particulières de mesurer la réfraction du porteur en vision de loin, par exemple à travers une fenêtre transparente permettant de voir droit devant et aussi en vision de près stimulée par une cible générée par l'appareil. Un tel appareil nécessite une mentonnière et un réglage à la fois de la mentonnière et de la tête de mesure de l'appareil. Toutefois, un auto-réfractomètre est limité à une mesure dans une posture de tête contrainte par la mentonnière et pour un angle de vision généralement horizontal. Un autoréfractomètre ne permet pas de mesurer la réfraction pour tous les comportements visuels, en particulier pour un port de tête différent. De plus, un autoréfractomètre est volumineux. Un autoréfractomètre permet généralement de mesurer certains paramètres tels que les écarts pupillaires (PD). Cependant, un autoréfractomètre ne permet pas de mesurer d'autres paramètres de monturisation essentiels tels que les demi-écarts la hauteur (H), ni de l'angle pantoscopique, la distance verre-oeil (DVO), ou encore la position du centre de rotation de l'oeil (CRO).

Les appareils de réfractométrie imposent généralement des contraintes au porteur de lunettes par l'utilisation d'une mentonnière, d'un appui frontal ou par le guidage du regard vers une cible dans un appareil ou encore par la contrainte d'un regard monoculaire. Ces contraintes posturales et/ou visuelles ne permettent pas d'effectuer des mesures de réfraction oculaire dans des conditions représentatives d'une vision naturelle binoculaire. De plus, ces appareils ne permettent pas de mesurer la réfraction oculaire du sujet pour différents ports de tête représentatifs de différents types de comportement visuel, tels que la vision de loin avec regard droit devant et la vision en lecture rapprochée avec regard abaissé.

Or, les caractéristiques de réfraction oculaire varient notamment en fonction de la posture, des conditions en vision de loin, en vision de près, en vision intermédiaire, de la direction du regard monoculaire ou binoculaire.

Il existe aussi des appareils plus mobiles, tels les skiascopes ou appareils de photoréfraction. Ces appareils ne nécessitent pas de mentonnière et permettent au futur porteur de lunettes d'adopter un comportement naturel et une posture quelconque. Néanmoins, ces appareils nécessitent de la part de l'opticien une certaine dextérité pour mesurer les paramètres de réfraction oculaire du porteur pour différentes postures. En effet, l'appareil doit être pratiquement aligné avec l'axe visuel en fonction du port de tête. Enfin, ces appareils ne sont pas aptes à mesurer des paramètres de monturisation (PD/H/DVO/CRO).

Un exemple d'un dispositif pour déterminer la réfraction sans mentonnière est le document US2003/0108350-A1. D'autres exemples sont les dispositifs des documents EP0305238-A1, FR2914173-A1 ou FR2952517-A1. D'autre part, la mesure de l'ensemble des paramètres de monturisation des lentilles de compensation sur une monture de lunettes pour un porteur nécessite un autre appareil de mesure et des étapes supplémentaires de mesure. La durée nécessaire pour effectuer l'ensemble des mesures de réfraction oculaire et des paramètres de monturisation nuit à l'efficacité d'une mesure globale du porteur ainsi qu'à la précision de la mesure, les conditions de mesure pouvant être différentes entre la mesure de réfraction et la mesure des paramètres de monturisation. En outre, l'utilisation de deux appareils différents est plus longue, plus complexe et nécessite davantage d'expertise de la part de l'opérateur. A l'usage, l'utilisation de deux appareils est finalement coûteuse.

OBJET DE L'INVENTION

Un des buts de la présente invention est de proposer un dispositif et un procédé de mesure des caractéristiques de réfraction oculaire objective d'un individu de manière précise, rapide et fiable dans différentes conditions de vision déterminées, par exemple en vision de loin (VL) et en vision de près (VP) et dans une posture non contraignante pour l'individu. Un autre but de l'invention est de proposer un dispositif et un procédé permettant de regrouper les mesures de réfraction oculaire objective et les mesures des paramètres de monturisation, afin que ces deux types de mesure soient effectués dans les mêmes conditions de vision et de posture de l'individu.

Afin de remédier aux inconvénients précités de l'état de la technique, la présente invention propose un dispositif de mesure binoculaire d'au moins une caractéristique de réfraction oculaire objective d'un sujet pour une pluralité de distances de vision. Plus particulièrement, selon l'invention ledit dispositif comprend :

- un système optique de visée de proximité variable apte à générer sélectivement une première cible ayant une première valeur de proximité P1 et au moins une deuxième cible ayant une deuxième valeur de proximité P2, ladite première cible et ladite deuxième cible étant centrées sur un seul et même axe optique de visée,
- au moins une source lumineuse apte à générer au moins un faisceau d'éclairage en direction des deux yeux du sujet,
- un appareil de capture d'images, ledit appareil ayant un axe optique de mesure aligné sur un axe de regard du sujet, ledit appareil étant adapté pour recevoir un faisceau de réfraction oculaire par réfraction du au moins un faisceau d'éclairage sur les yeux du sujet, l'appareil de capture d'images étant adapté pour capturer une première image de réfraction oculaire des deux yeux lorsque la première cible de proximité P1 est activée et au moins une deuxième image de réfraction oculaire des deux yeux lorsque la deuxième cible de proximité P2 est activée, et
- un calculateur adapté pour recevoir la première image de réfraction oculaire et la deuxième image de réfraction oculaire pour en déduire une mesure d'au moins une caractéristique de réfraction oculaire objective des deux yeux du sujet en fonction de la première valeur de proximité P1 et de la deuxième valeur de proximité P2,
- un boîtier supportant le système optique de visée, la au moins une source lumineuse et l'appareil de capture d'images,
- l'axe optique de visée est incliné d'un angle alpha par rapport à l'horizontale, l'angle alpha étant compris entre +5 degrés et +85 degrés lorsque le boîtier est posé sur une surface horizontale, et
- l'appareil de capture d'images et le calculateur étant adaptés pour mesurer des écarts pupillaires pour la première valeur de proximité P1 et pour la deuxième valeur de proximité P2 et/ou au moins un paramètre de monturisation parmi la hauteur, l'angle pantoscopique, la distance verre-oeil et la position du centre de rotation de l'oeil.

Le dispositif de l'invention permet de réaliser des mesures de réfraction pour une pluralité de distances de vision dans au moins une posture de vision où le regard du sujet est incliné par rapport à une ligne horizontale. Le dispositif de l'invention permet notamment une mesure de réfraction en vision de près dans une posture naturelle de vision sans contrainte. Le dispositif de l'invention permet de varier la valeur de proximité de la cible et de mesurer la réfraction pour une autre valeur de proximité sans modifier l'axe de visée qui reste incliné par rapport à une ligne horizontale.

De préférence, l'angle alpha est compris entre +15 degrés et +40 degrés.

De façon avantageuse, le dispositif de mesure comprend des moyens de déplacement et/ou d'orientation de l'axe optique de visée de manière à aligner l'axe optique de mesure sur l'axe du regard du sujet.

Selon un mode de réalisation particulier, le dispositif comprend en outre des moyens de mesure de distance entre ledit dispositif et la tête du sujet, lesdits moyens de mesure de distance étant choisis parmi : un télémètre, un système de traitement d'image basé sur la qualité image, un système de traitement d'image basé sur la mesure de repères montés sur un clip fixé à une monture de lunettes, un système d'étalonnage ou un système de mesure de distance par ultrasons.

Avantageusement, la au moins une source lumineuse comprend au moins une source infrarouge et l'appareil de capture d'images est apte à capturer des images dans l'infrarouge.

Selon un mode de réalisation particulier, le dispositif comprend en outre un séparateur optique de faisceau disposé sur le trajet optique entre le sujet et la au moins une source, l'appareil de capture d'images, le séparateur optique de faisceau étant apte à combiner le faisceau d'éclairage et la première cible ou la deuxième cible sur l'axe optique de visée en direction des yeux du sujet, ledit séparateur de faisceau étant apte à diriger le faisceau de réfraction oculaire en direction de l'appareil de capture d'images sur l'axe optique de mesure.

Avantageusement, le séparateur optique de faisceau comporte un miroir dichroïque.

Préférentiellement, le séparateur optique de faisceau comporte un miroir dichroïque apte à transmettre le faisceau d'éclairage infrarouge et à réfléchir le faisceau cible.

De façon avantageuse, l'angle entre l'axe optique de mesure et l'axe optique de visée est inférieur à 10 degrés.

Préférentiellement, les axes optiques de mesure et de visée sont confondus.

Selon un mode de réalisation particulier, l'appareil de capture d'images est adapté pour former une image du visage du sujet sur un champ objet ayant un diamètre d'au moins 50 mm.

L'invention concerne aussi un procédé de mesure binoculaire d'au moins une caractéristique de réfraction oculaire objective d'un sujet pour une pluralité de distances de vision, ledit procédé comprenant les étapes suivantes :

- génération d'une première cible ayant une première valeur de proximité P1 suivant un axe optique de visée alignée avec l'axe du regard du sujet, l'axe du regard étant incliné d'un angle alpha1 compris entre +5 degrés et +85 degrés par rapport à l'horizontale ;
- capture d'une première image de réfraction oculaire des deux yeux suivant une direction de mesure lorsque la première cible de proximité P1 est activée;
- génération d'une deuxième cible ayant une deuxième valeur de proximité P2 suivant le même axe optique de visée destiné à être alignée avec l'axe du regard du sujet, l'axe du regard étant incliné d'un angle alpha2 compris entre +5 degrés et +85 degrés par rapport à l'horizontale ;
- capture d'au moins une deuxième image de réfraction oculaire des deux yeux suivant la même direction de mesure lorsque la deuxième cible de proximité P2 est activée ;
- traitement numérique de la première image de réfraction oculaire et de la au moins une deuxième image de réfraction oculaire pour en déduire au moins une caractéristique de réfraction oculaire objective des deux yeux du sujet en fonction de la première valeur de proximité P1 et de la deuxième valeur de proximité P2,

le procédé comprenant en outre une étape de mesure des écarts pupillaires pour la première valeur de proximité P1 et pour la deuxième valeur de proximité P2 et/ou au moins un paramètre de monturisation parmi la hauteur, l'angle pantoscopique, la distance verre-oeil et la position du centre de rotation de l'oeil.

Selon un aspect préféré du procédé de l'invention, la capture d'une première image de réfraction oculaire est effectuée pour une première posture du sujet, la capture d'une deuxième image de réfraction oculaire est effectuée pour une deuxième posture du sujet différente de la première posture et les étapes de capture d'images sont réalisées dans des conditions où la tête du sujet est libre de contraintes physiques extérieures.

Dans un mode de réalisation particulier, le procédé comprend en outre une étape préliminaire de mise en place de manière à positionner et orienter le dispositif de mesure relativement au sujet.

Avantageusement, le procédé de mesure comprend une étape d'alerte en cas de défaut de positionnement relatif ou d'orientation relative entre le dispositif de mesure et le sujet.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 représente schématiquement un appareil de mesure selon un mode de réalisation de l'invention, posé sur une table face à un porteur ;
- la figure 2a est une vue de côté d'un appareil de mesure selon un mode de réalisation de l'invention, en vision de loin ;
- la figure 2b est une vue de côté de l'appareil de mesure de la figure 2a en vision de près ;
- la figure 3 est une vue en perspective d'un boîtier de mesure d'un appareil de mesure selon un mode de réalisation de l'invention ;
- la figure 4 est une image d'un porteur équipé d'un clip avec repères ;
- la figure 5 représente un exemple d'interface graphique d'un appareil de mesure selon un mode de réalisation de l'invention ;
- la figure 6 illustre schématiquement une image d'un porteur de lunettes sur laquelle est représentée une cartographie des paramètres de réfraction oculaire mesurés dans différentes conditions de vision et/ou de posture.

Dispositif

On propose un dispositif permettant de réaliser des mesures de réfraction oculaire sans contrainte sur la tête du porteur, c'est-à-dire sans mentonnière et sans appui frontal. Le dispositif laisse donc le porteur libre de son port de tête. Le dispositif permet de réaliser des mesures de réfraction oculaire objective pour différents comportements de vision, y compris différentes postures de tête. Optionnellement, le dispositif de l'invention permet aussi de réaliser des mesures de paramètres de monturisation. Le dispositif est à la fois compact et simple d'utilisation.

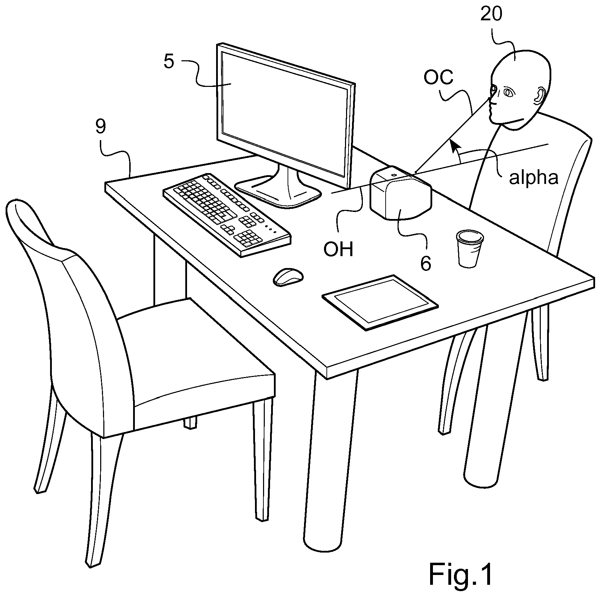

Sur la figure 1, on a représenté schématiquement un dispositif selon un premier mode de réalisation de l'invention, dans des conditions d'utilisation où le dispositif est posé sur une table face à un porteur assis. Le dispositif de la figure 1 comporte :

- un boîtier 6 qui intègre un capteur d'images (par exemple une caméra), un système d'éclairage pour éclairer le visage 20 du porteur (notamment les deux yeux) et un système optique de visée ayant une proximité variable entre au moins deux valeurs différentes de proximité, par exemple en vision de loin et en vision de près ;
- un calculateur permettant d'extraire des images prises par la caméra les valeurs de réfractions du porteur et optionnellement les mesures des paramètres de monturisation ;
- un système de contrôle de mesure et d'affichage des résultats de mesure.

De façon avantageuse, un ordinateur muni d'une interface graphique, par exemple un écran de visualisation 5, réalise les fonctions du calculateur, du système de contrôle et d'affichage des résultats de mesure.

Le boîtier 6 et l'écran de visualisation 5 sont posés sur une table 9. Le boîtier 6 est disposé face au visage 20 du porteur de manière à permettre l'éclairage du visage 20 du porteur et l'acquisition d'images d'au moins une partie du visage du porteur et en particulier des deux yeux. Avantageusement, le capteur d'image comprend une caméra, fonctionnant de préférence dans l'infrarouge. La caméra filme le futur porteur de lunette. Plus précisément, la caméra vise le visage 20 du porteur à une distance comprise entre 300 et 700mm, la mise au point de l'image étant faite sur le visage. Avantageusement, le champ de la caméra permet de visualiser à la fois les yeux du porteur et une grande partie de son visage, notamment autour des yeux.

Si le porteur est équipé d'une monture de lunettes 7 (sa monture avec correction ou une monture choisie), le champ de la caméra permet de voir cette monture en grande partie, et de préférence en intégralité.

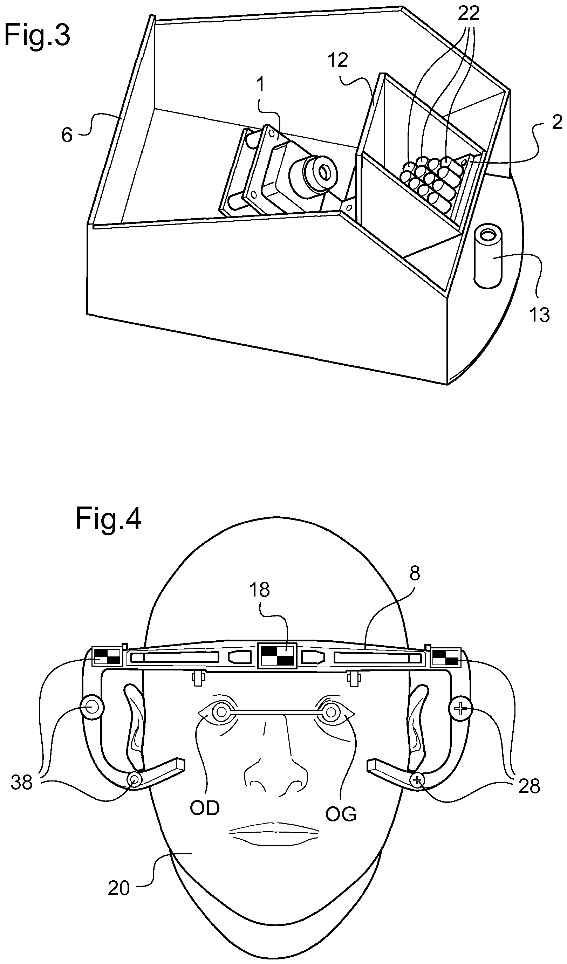

Si le porteur est équipé d'une monture avec un clip 8 muni de repères 18, 28, 38 (voir figure 4) ou d'une monture spéciale équipée de repères, ces repères sont visibles par la caméra, c'est-à-dire que le champ de la caméra est compatible avec la position des repères par rapport aux yeux, et les repères possèdent un contraste suffisant dans l'infrarouge pour pouvoir être automatiquement détectés.

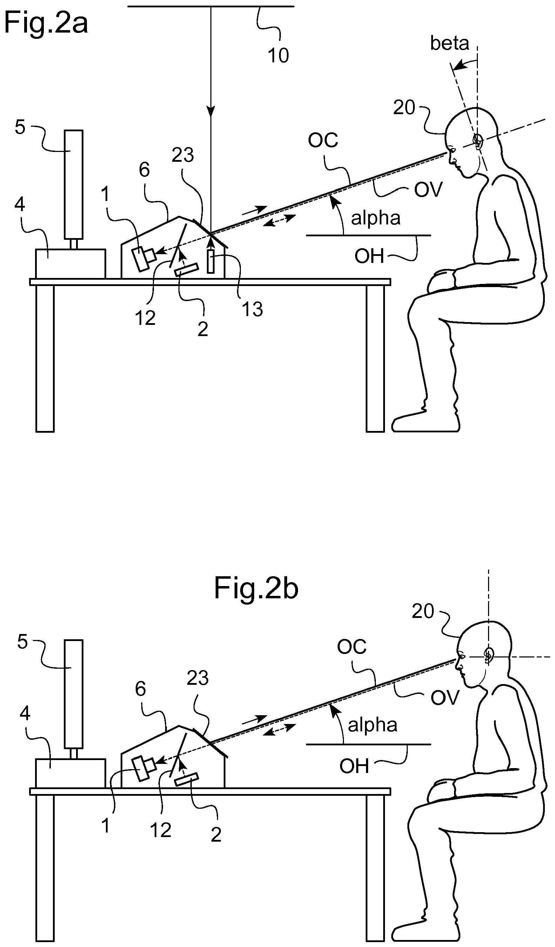

La caméra filme le porteur avec un certain angle alpha non nul par rapport à l'horizontale. De préférence, le dispositif est positionné sur une table 9, par exemple une table de vente de l'opticien, la table étant à une hauteur inférieure à celle du visage 20 du porteur. L'angle de visée de la caméra est ainsi incliné d'un angle alpha positif (voir figures 1 et 2).

Sur les figures 2a et 2b, on a représenté une vue en coupe du dispositif incluant le boîtier 6 du dispositif de la figure 1.

Le boîtier 6 des figures 2a-2b comporte :

- une caméra 1, ayant de préférence un capteur d'images infrarouge, et permettant de filmer le visage du porteur ; l'axe optique OC de la caméra 1 est disposé de manière à viser l'axe du regard du porteur ; l'axe optique OC forme un angle alpha positif avec une droite horizontale OH ;
- un système d'éclairage 2, de préférence infrarouge, est utilisé pour l'éclairage des yeux du porteur et pour réaliser les mesures de réfraction par skiascopie ou par photoréfraction ;
- un séparateur de faisceau 12 disposé sur le chemin optique entre la caméra 1 et le système d'éclairage 2 pour combiner le faisceau d'éclairage sur l'axe optique de la caméra ;

- un système optique de visée permettant au porteur de basculer d'une vision à une distance prédéterminée à une autre distance de vision différente, tout en conservant une direction de regard identique ;

- un ordinateur 4 muni d'un écran de visualisation 5, pour contrôler la commande des éléments du boîtier et afficher les résultats de mesure de préférence sous forme graphique.

Sur les figures 2a et 2b, on a représenté en tirets le chemin optique des faisceaux infrarouges et en trait continu le chemin optique des faisceaux dans le domaine visible. Le séparateur de faisceau 12 est orienté de manière à diriger le faisceau infrarouge d'éclairage généré par le système d'éclairage 2 vers une ouverture dans le boîtier 6 en direction du visage 20 du porteur. Par réflexion sur le visage et les yeux du porteur, un faisceau infrarouge est renvoyé en direction du boîtier. Le séparateur de faisceau 12 reçoit le faisceau infrarouge rétro-réfléchi et le transmet en direction de la caméra 1. La caméra 1, le système d'éclairage 2 et le séparateur de faisceau 12 sont disposés les uns par rapport aux autres de manière à ce que l'axe optique OC de la caméra 1 forme un angle alpha avec une droite horizontale OH et de manière à ce que le faisceau d'éclairage généré par le système d'éclairage soit centré sur l'axe optique OC.

De façon particulièrement avantageuse, l'axe optique OC du capteur d'images est confondu avec l'axe optique du faisceau d'éclairage. L'axe optique OC est dirigé vers le visage du porteur de manière à ce que, le porteur étant dans une posture de vision non contrainte, l'axe de son regard soit de préférence centré sur l'axe optique OC

Le dispositif de mesure comporte en outre avant de la caméra et du système d'éclairage, un système optique de visée permettant de faire varier la proximité d'observation vue par le porteur, typiquement entre -0.5 et +10 Dioptries.

Préférentiellement, comme représenté sur la figure 2a, en vision de loin, on utilise un miroir plan 23 particulier, appelé miroir froid. Ce miroir 23 a la particularité d'être transparent dans l'infrarouge et réfléchissant dans le visible. La caméra 1 acquiert des images à travers le miroir 23 comme à travers une fenêtre transparente. Le miroir 23 peut être utilisé conjointement avec une source lumineuse 13 émettant un rayonnement dans le visible (par exemple dans le vert) afin de présenter au porteur une image au loin. Alternativement, le miroir 23 peut être utilisé sans source 13. A titre d'illustration, le miroir 23 peut être orienté de manière à ce que le porteur visualise le plafond 10 du magasin de l'opticien. Lorsque la source 13 est présente et allumée, celle-ci génère au plafond 10, un motif lumineux que le porteur peut voir par réflexion sur le miroir 23. La zone de plafond destinée à être éclairée par la source 13 sera préférentiellement dépourvue d'éclairage et possédera une surface diffusante.

Sur la figure 2a, lorsque la source 13 est éteinte, le porteur regarde directement le plafond 10. Le porteur visualise ainsi une scène en condition de proximité proche de 0 Dioptrie. En alternative, pour que le porteur visualise en condition de vision de loin, on peut utiliser une inclinaison de miroir 23 telle que le porteur visualise un objet réellement situé en vision de loin (objet à l'extérieur du magasin par exemple), l'axe de son regard étant aligné sur l'axe optique OC de la caméra. La tête du porteur est inclinée d'un angle beta par rapport à la verticale de manière à simuler la posture de vision de loin avec l'axe du regard droit devant. Dans ces conditions, la caméra permet simultanément de filmer le visage du porteur et de mesurer la réfraction en vision de loin.

Alternativement, un miroir mobile en translation permet de basculer d'une cible en vision de loin à une cible en vision de près.

Selon un mode de réalisation particulier, représenté sur la figure 2b, le miroir 23 porte une sérigraphie sur sa surface afin de proposer un motif pour stimuler la vision de près (à environ 400mm du visage du porteur). Ainsi, pour réaliser la mesure de réfraction en vision de près, on éteint la source 13 (si celle-ci est présente) et on demande au porteur de regarder le motif sérigraphié sur la surface du miroir 23. La tête du porteur est ici droite et l'axe du regard est incliné par rapport à une ligne horizontale de manière à simuler la posture de vision de près avec des verres progressifs. Dans ces conditions, la caméra permet simultanément de filmer le visage du porteur et de mesurer la réfraction en vision de près.

En alternative, pour la distance en vision de loin on peut utiliser un miroir froid concave, dont le foyer correspond environ à 400mm (le foyer coïncide avec le visage du porteur). Dans ce cas, on utilise directement le visage du porteur qui est ramené à l'infini par le miroir froid concave pour la mesure en vision de loin. Pour la mesure en vision de près, on commute par exemple le miroir de manière à visualiser une autre cible.

En alternative pour la vision de près, on utilise une source lumineuse placée à proximité du miroir et à proximité de l'axe de la caméra, ou on utilise un masque sérigraphié opaque dans le visible et transparent dans l'IR que l'on superpose au miroir (le porteur n'est ainsi pas gêné par le reflet du miroir pour faire la mise au point en vision de près) ou un masque percé, le trou servant de point de fixation en vision de près.

Les sérigraphies utilisées peuvent être réalisées avec des encres transparentes dans l'infrarouge ou fluorescentes dans le visible afin de ne pas impacter la mesure.

La Figure 3 représente une vue en perspective d'un boîtier d'un appareil de mesure selon un mode de réalisation de l'invention. La figure 3 permet de détailler le système d'éclairage et de capture d'images.

Le système d'éclairage 2, préférentiellement infrarouge, est utilisé pour réaliser les mesures de photoréfraction. De façon avantageuse, le système d'éclairage comprend plusieurs secteurs de diodes électroluminescentes (Led) infrarouges. Les Leds 22 éclairent le visage du porteur et génèrent sur la rétine de l'oeil une tache dont la taille dépend de l'amétropie du porteur.

La caméra 1 permet de visualiser l'intensité lumineuse liée à la diffusion de la tache sur la rétine, et on perçoit alors au niveau de la pupille de l'oeil un gradient d'intensité lié à l'amétropie des yeux (principe de photoréfraction bien connu).

L'utilisation de différents secteurs de Leds 22 permet de mesurer l'amétropie des yeux pour différents axes et d'en déduire des paramètres d'amétropies sphérico-cylindriques.

Les différents secteurs de Leds sont allumés alternativement et les images sont prises de manière synchrone avec l'allumage d'un secteur particulier afin de réaliser la mesure de réfraction pour cet axe. On observe alors pour les pupilles des yeux des gradients d'intensité variables en cas de présence d'astigmatisme.

Les différents secteurs de Leds 22 réalisent un motif dont le centre coïncide avec l'axe optique OC de la caméra 1 infrarouge. Dans le schéma des figures 2a-2b et 3, on utilise une lame semi-transparente 12 dans l'infrarouge afin de réaliser cette coïncidence. De manière alternative, on peut aussi disposer les Leds 22 tout autour de l'objectif de la caméra 1.

Un calculateur, représenté sur les figures 1 et 2a-2b par un ordinateur 4, stocke les différentes images et en extrait (par mesure du gradient d'intensité dans les pupilles des yeux suivant la source utilisée) les valeurs d'amétropie pour chacun des deux yeux et pour une condition de vision et un comportement visuel particulier.

L'écran 5 de l'ordinateur 6 est utilisé afin de présenter les résultats de la mesure. L'écran 5 permet aussi le contrôle de la mesure, en particulier pour s'assurer que les yeux et le visage sont bien dans le champ de mesure de la caméra.

L'opticien peut déplacer le dispositif de mesure et l'orienter (cas d'un porteur grand/petit) afin de replacer le visage et les yeux dans le champ de mesure. Il suffit pour cela de déplacer et/ou d'orienter le boîtier 6.

Avantageusement, le boîtier 6 est équipé de patins glissants afin de permettre à l'opticien de le déplacer facilement sur la table 9. Il est éventuellement équipé d'un réglage en orientation afin de permettre la visualisation du visage de porteur de très grande ou très petite taille.

En option (Figure 4), le dispositif comprend un clip 8 destiné à être placé sur la monture 7 de lunettes du porteur. Le clip 8 est monté sur la monture 7 du porteur (monture choisie ou monture actuelle), ou éventuellement sur une monture spéciale utilisée uniquement pour la mesure. Ce clip 8 permet de déterminer précisément la distance entre le porteur et le dispositif, de mesurer précisément la réfraction, et de déduire les paramètres de monturisation (PD/H/DVO...) s'il est utilisé avec la monture choisie. Le clip 8 est utilisé pour réaliser les mesures de paramètres de monturisation et/ou pour permettre de contrôler précisément le port de tête du porteur et pour affiner les mesures de réfraction réalisées.

En alternative du clip 8, on peut utiliser tout système permettant de mesurer la position de la tête par rapport à l'appareil de mesure (par exemple : on mesure la distance à l'aide d'un télémètre optique ou à ultrasons). On mesure la position de la tête en utilisant la monture comme référence, les paramètres géométriques de la monture étant parfaitement connus par ailleurs.

En alternative à un boîtier 6 posé sur une table 9, le dispositif de l'invention peut être du type nomade (par exemple tablette-PC), le dispositif comportant un appareil tenu à la main par le porteur lui-même. Avantageusement, la mesure en VP est effectuée l'appareil étant posé sur une table et la mesure en VL est réalisée l'appareil étant tenu à la main. Cette solution risque toutefois de générer des pertes de précision de mesure dues à des tremblements lors de la mesure.

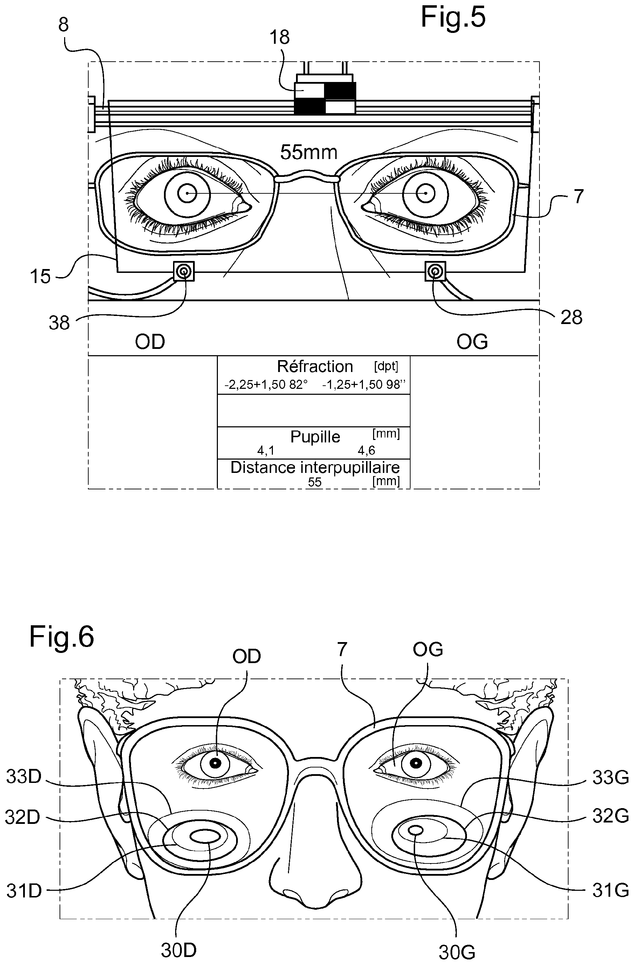

La figure 5 représente un exemple d'interface graphique d'un appareil de mesure selon un mode de réalisation de l'invention, après réglage correct à l'étape 2 du procédé, la partie supérieure représente une image du visage du porteur sur laquelle sont affichés des paramètres de mesure de réfraction oculaire et des paramètres de monturisation et la partie inférieure de l'image indique d'autres mesures de paramètres de réfraction oculaire du porteur et de paramètres de monturisation. Sur l'image vidéo de la partie supérieure, un rectangle 15 de cadrage est affiché en superposition sur l'image du visage du porteur. Ce rectangle 15 sert de repère pour contrôler le centrage des yeux du porteur par rapport au champ de vision de la caméra.

Les avantages de ce dispositif sont les suivants :

- compatible avec une utilisation sur table, car très compact ;
- pas de contraintes sur le porteur (pas de mentonnière ni d'appui frontal) ;
- possibilité de mesurer la réfraction pour au moins deux valeurs de proximité et une posture de tête quelconque ;
- capacité à mesurer en même temps les paramètres de monturisation (via l'utilisation d'un clip) ;
- capacité à mesurer plus précisément la réfraction (si utilisation du clip).

**Procédé**

On propose aussi une méthode de mesure de la réfraction pour différentes postures/comportements visuels, cette méthode comportant les étapes suivantes :

- mesure de la réfraction pour une première distance de vision prédéterminée et dans une première posture prédéterminée. Le contrôle de la posture est réalisé via l'affichage sur l'écran ou optionnellement par utilisation du clip. Par exemple, l'axe du regard est sensiblement abaissé et la proximité de la première distance de vision correspond à la vision de près (Fig. 2b).
- mesure de la réfraction pour une deuxième distance de vision différente de la première distance de vision, dans une deuxième posture de tête différente de la première posture de tête. Ici aussi, la nouvelle posture est contrôlée via l'affichage sur l'écran ou optionnellement par utilisation du clip. Par exemple, la tête est inclinée vers l'avant (Fig. 2a). Lors de cette deuxième mesure, l'axe de regard est sensiblement identique à celui de la première mesure, mais la posture de tête et/ou la distance de vision sont différentes.

De façon avantageuse, on utilise le dispositif en lien avec les figures 2a-2b, de la manière suivante :
Le dispositif est placé sur la table de vente de l'opticien. Le dispositif est connecté à un ordinateur qui sert à la fois de calculateur et de système d'interface graphique d'affichage et de contrôle. L'opticien est en face de son ordinateur et le porteur est assis en face de lui. On suppose ici que le porteur est équipé de la monture choisie, elle-même équipée d'un clip.

Etape 1 : réglage du dispositif en position

L'opticien démarre le dispositif, et une image vidéo apparaît sur l'écran de l'ordinateur, fournissant l'image capturée par la caméra infrarouge (IR). Afin d'obtenir une image suffisamment lumineuse, l'ensemble des LEDs IR peut être allumé à ce moment.

L'opticien demande alors au porteur de regarder une première cible générée par le dispositif (par exemple, la projection au plafond de la source 13 à travers le miroir 23). L'opticien règle alors le dispositif afin que les yeux du porteur et le visage soit dans le champ de la caméra, en déplaçant le boîtier 6 sur la table.

Eventuellement, si le porteur est de taille notablement différente de la moyenne (très petit ou très grand), l'opticien peut être amené à régler l'angle alpha pour recentrer le visage dans le champ de la caméra. De manière alternative, on peut régler la hauteur du porteur (chaise réglable) afin de positionner le visage dans le champ de la caméra.

Un réglage de distance entre la caméra et le visage du porteur peut aussi s'avérer nécessaire. Dans ce cas, l'opticien règle la distance de manière à avoir une image nette à l'écran. Si le clip 8 est utilisé, on peut aussi utiliser les repères du clip afin de déterminer précisément cette distance et indiquer à l'opticien le sens du réglage. Pendant cette étape de réglage, la tête du porteur reste libre de ses mouvements sans contrainte.

Les étapes qui suivent sont décrites dans un ordre qui peut être permuté.

Etape 2 : réglage du port de tête en vision de loin

Le porteur fixe ici la première cible en vision de loin, par exemple la cible projetée au plafond par la source 13. L'opticien peut éventuellement demander au porteur de pivoter la tête vers l'avant ou vers l'arrière, tout en continuant à fixer la cible, si on estime que la tête est trop inclinée ou si on souhaite modifier le port de tête.

Par exemple, si les pupilles des yeux ne sont pas centrées dans les montures, on peut demander au porteur d'incliner la tête pour les recentrer. Ceci permet alors d'avoir une posture représentative d'une posture en vision de loin.

De façon alternative, on peut utiliser l'indication d'angle pantoscopique fournie par les repères du clip pour contrôler le port de tête, par exemple en vérifiant que l'angle pantoscopique mesuré est proche de l'angle pantoscopique de la monture.

De manière générale, l'opticien peut demander au porteur d'adopter un port de tête qu'il juge utile à la mesure de réfraction.

Quand l'appareil est bien positionné, une image équivalente à celle présentée en figure 5 s'affiche sur l'écran. Ici, le port de tête est tel que l'abaissement du regard par rapport à la tête est à peu près nul (proche d'un abaissement du regard par rapport à la tête en vision de loin).

Une mesure peut alors être réalisée, et les différents secteurs de LED IR sont allumés suivant une séquence prédéterminée afin de réaliser une première mesure de réfraction. La valeur de réfraction de chaque oeil est alors affichée en bas de l'écran de visualisation tel que représenté sur la figure 5, pour une première posture et pour la première distance de vision, ou dit autrement pour la première valeur de proximité P1.

On peut contrôler que le porteur regarde bien la cible au plafond 10 en vérifiant que son regard converge sur la cible au plafond, par exemple en mesurant la position relative des pupilles et des reflets cornéens.

En pratique, pour réaliser la mesure, on procède comme suit :

Quand on considère que le porteur est bien positionné, on déclenche la mesure (par exemple par appui sur un bouton) et on récupère une séquence d'images prise avec les différentes Leds IR.

La séquence d'images est enregistrée sur l'ordinateur 4 pour traitement subséquent.

Une de ces images peut par exemple être prise avec toutes les Leds IR allumées, ce qui permet d'avoir un flux lumineux maximal. Sur cette image, on repère la position des pupilles, la position des reflets cornéens, et la position des différents marqueurs du clip.

La position des reflets cornéens ou des centres des pupilles permet de déterminer la distance interpupillaire (PD) en nombre de pixels sur l'image, et la distance en pixels entre les marqueurs du clip permet de connaître la taille d'un pixel en mm. On peut ainsi déterminer la valeur de l'écart pupillaire en mm.

De même, on peut mesurer la distance entre les reflets cornéens/centres de pupille et les bords inférieurs de la monture pour déterminer les hauteurs de montage, ou la distance entre les reflets cornéens/centres de pupille et le milieu du pont pour déterminer les ½ écarts pupillaires.

Les autres images de la séquence peuvent être utilisées pour mesurer la réfraction, par exemple chaque image correspond à l'allumage d'un secteur de LEDs IR et donc à la mesure de l'amétropie pour différents axes.

On détermine sur chaque image la taille de la pupille et son intensité ou plus précisément son gradient d'intensité. Le sens du gradient et sa valeur renseigne directement sur l'amétropie de l'oeil concerné dans l'axe mesuré, et il suffit d'au moins trois images pour trois secteurs différents (par exemple à 0°, 120° et 240°) pour déterminer complètement la sphère, le cylindre et l'axe de cylindre de l'oeil. Il est possible d'utiliser plus de trois images pour améliorer la mesure.

Afin d'augmenter la dynamique de mesure, on peut utiliser des Leds d'excentricité croissante pour chaque secteur. Par exemple, si la Led d'excentricité minimale pour le premier secteur donne lieu à une saturation sur le gradient d'intensité de la pupille, on utilise alors l'image correspondant à la Led d'excentricité immédiatement supérieure, et ainsi de suite, jusqu'à ne plus observer de saturation. On considère qu'il y a saturation du gradient d'intensité dans la pupille quand celle-ci possède des zones où l'intensité, tout en étant forte, ne varie quasiment pas (zone en bord de pupille).

L'amétropie Ai pour le secteur i considéré est alors donnée par une fonction Fi qui dépend du diamètre pupillaire, du gradient et de l'excentricité :

$$Ai = Fi(Diam, Gradient, Ex).$$

Cette fonction Fi est obtenue par calibration préalable de l'appareil ou par calcul (cf. « Light-intensity distribution in eccentric photorefraction crescents", Kussel J. Opt. Soc. Am. A/ Vol. 15, No. 6/June 1998).

Etape 3 : mesure suivant une autre posture et/ou un autre comportement visuel

On demande cette fois-ci au porteur de regarder une deuxième cible en vision de près (cf Fig. 2b). Pour ce faire, la cible projetée au plafond via la source 13 est éteinte et le porteur regarde le motif sérigraphié sur le miroir 23.

Il n'est normalement pas nécessaire à ce stade de régler de nouveau la position et l'orientation du boîtier 6.

A ce stade, l'opticien peut demander au porteur de pivoter la tête afin de lui imposer une posture de tête adéquate. Par exemple, on peut demander au porteur de relever la tête, tout en fixant la cible sérigraphiée, jusqu'au moment où les pupilles se situent en bord inférieur de monture, ce qui correspond à une zone de vision en vision de près pour un verre progressif.

En alternative, on peut utiliser la mesure de l'angle pantoscopique via les repères du clip pour contrôler ou imposer une posture de tête déterminée, par exemple pour imposer une posture de tête générant un abaissement de regard de 30 degrés par rapport à la vision de loin.

Dans ce cas, on affiche par exemple sur l'écran 5, l'angle de rotation de la tête entre la première posture (en vision de loin) et la deuxième posture (en vision de près) afin d'aider l'opticien à choisir la posture qui convient.

On peut aussi contrôler que le porteur regarde bien la cible sérigraphiée en vérifiant que son regard converge sur la cible, par exemple en mesurant la position relative des pupilles par rapport aux reflets cornéens.

L'ensemble de ces mesures (étape 1 et étape 2) peut être fait de manière continue et on peut mesurer la réfraction pour un ensemble continu de posture de tête pour chacune des étapes. A partir de cet ensemble de mesures, on peut afficher sur une image représentant la monture, la réfraction mesurée aux points d'intersection du regard et du plan de la monture pour l'oeil droit et l'oeil gauche.

A titre d'exemple illustratif, la figure 6 représente une image de cartographie des réfractions en projection dans le plan de la monture. Plus précisément, la figure 6 représente schématiquement une image d'un porteur de lunettes sur laquelle est représentée une cartographie des paramètres de réfraction oculaire mesurés dans différentes conditions de vision et affichés en projection dans le plan des lentilles de correction. Les courbes 30G, 31G, 32G, 33G, 30D, 31D, 32D, 33D représentent des exemples de courbes isosphères pour la vision de près, par pas de 0.125 dioptrie entre deux courbes adjacentes, chaque courbe étant centrée sur le point d'intersection du regard de chaque oeil avec le plan de la monture.

De plus, les mesures de réfraction peuvent être rendues plus précises grâce à l'utilisation du clip 8. En effet, la photoréfraction se fait généralement sans mesure de distance précise, ce qui conduit à des imprécisions sur les mesures de réfraction. Connaissant les paramètres du clip, par des procédures de calibration ou d'étalonnage, on peut ainsi, connaissant la distance précise entre le dispositif et le porteur, mesurer précisément diamètres pupillaires ainsi que la distance entre la pupille de l'oeil et la caméra. Or, ces deux paramètres sont très importants pour la précision de la mesure. En effet, le gradient d'intensité est proportionnel au carré de diamètre pupillaire. Une mesure imprécise du diamètre pupillaire génère par conséquent une erreur importante sur la mesure de réfraction.

On peut aussi utiliser des montures équipées de verres correcteurs (pré-montées ou lunette d'essai) afin de corriger au moins partiellement l'amétropie en vision de loin du porteur. Ceci permet au porteur d'avoir une vision relativement nette lors des étapes 2 et 3. L'utilisation de verres de puissance non nulle pendant la mesure, permet aussi de limiter la dynamique de mesure du dispositif et favorise ainsi la précision de mesure. Dans ce cas, en pratique, on veille à ce que les reflets des surfaces des verres ne se superposent pas aux pupilles des yeux.

On réalise de plus un correctif sur la mesure en prenant en compte la perte de lumière liée aux réflexions sur les surfaces (4% environ par surface, aller et retour) et on déduit de la mesure la puissance du verre.

Etape 4 (optionnelle) mesure des PD/H/DVO/CRO

On peut, lors de l'étape 2 ou l'étape 3, mesurer les écarts pupillaires en vision de loin et en vision de près. Pour ce faire, on utilise à la fois les repères 18, 28, 38 du clip 8 pour réaliser une mise à l'échelle de l'image, les reflets cornéens ou le cercle pupillaire et les contours de la monture 7 visualisés grâce à l'éclairage infrarouge.

L'éclairage infrarouge présente l'avantage de générer un très bon contraste sur les pupilles et de ne pas aveugler le porteur.

La mesure du diamètre pupillaire ou du demi-diamètre pupillaire se fait par la mesure du centre des pupilles (ou reflets cornéens) et par la détermination du centre de la monture.

La mesure des hauteurs (H) se fait par la mesure de la distance entre les centres des pupilles et les bords inférieurs droit et gauche de la monture 7. Le port de tête impactant fortement la mesure des hauteurs, on peut envisager une mesure en deux étapes afin d'améliorer la précision de cette mesure. Dans ce cas, une mesure est réalisée en posture naturelle (le porteur regarde droit devant) et une deuxième mesure est réalisée, cette deuxième mesure étant l'étape 2 ou l'étape 3. Le dispositif est dans ce cas équipé d'un inclinomètre afin de réaliser la correction de posture.

La mesure de la distance verre-oeil (DVO) se fait par une analyse des disparités entre les deux postures naturelles aux étapes 2 ou 3.

Les avantages de la méthode de mesure de réfraction de l'invention sont les suivantes :

- réalisation de mesures de réfraction pour des postures de tête variables ;
- mesure au préalable des postures de têtes correspondant à des postures naturelles et ensuite mesure de réfaction dans ces postures mesurées.
- variation de l'abaissement du regard par rapport à la tête de manière quelconque, en jouant sur la posture de tête, l'axe du regard restant fixe et centré sur l'axe de mesure, ce qui permet avantageusement de mesurer quasi-continuement la variation de réfraction en fonction de l'abaissement du regard par rapport à la tête. L'axe du regard restant fixe et incliné par rapport à l'horizontale, le porteur peut en effet modifier librement l'abaissement du regard par rapport à la tête simplement en abaissant ou relevant sa tête.
- possibilité de mesurer simultanément les écarts pupillaires pour différentes distances de vision, ainsi que des paramètres de monturisation tels que hauteur / angle pantoscopique /DVO (si utilisation d'un clip) et ce qui permet de limiter le nombre et la durée des mesures pour réaliser un verre personnalisé.

## Revendications

1. Dispositif de mesure binoculaire d'au moins une caractéristique de réfraction oculaire objective d'un sujet pour une pluralité de distances de vision, **caractérisé en ce que** ledit dispositif comprend :

   - un système optique de visée (13, 23) de proximité variable apte à générer sélectivement une première cible ayant une première valeur de proximité P1 et au moins une deuxième cible ayant une deuxième valeur de proximité P2, ladite première cible et ladite deuxième cible étant centrées sur un seul et même axe optique de visée (OV), permettant la variation d'un abaissement du regard du sujet par rapport à sa tête , tout en conservant un axe de regard identique,
   - au moins une source lumineuse (2) apte à générer au moins un faisceau d'éclairage en direction des deux yeux du sujet,

     - un appareil de capture d'images (1), ledit appareil ayant un axe optique de mesure (OC) aligné sur un axe de regard du sujet, l'appareil de capture d'images étant disposé et ayant un champ adapté pour l'acquisition d'image des deux yeux et d'au moins une partie du visage du porteur,
     - ledit appareil étant adapté pour recevoir un faisceau de réfraction oculaire par réfraction du au moins un faisceau d'éclairage sur les yeux du sujet, l'appareil de capture d'images étant adapté pour capturer une première image de réfraction oculaire des deux yeux lorsque la première cible de proximité P1 est activée et au moins une deuxième image de réfraction oculaire des deux yeux lorsque la deuxième cible de proximité P2 est activée et
     - un calculateur adapté pour recevoir la première image de réfraction oculaire et la deuxième image de réfraction oculaire pour en déduire une mesure d'au moins une caractéristique de réfraction oculaire objective des deux yeux du sujet en fonction de la première valeur de proximité P1 et de la deuxième valeur de proximité P2, et en fonction d'un abaissement du regard par rapport à la tête du sujet, tout en maintenant l'axe du regard centré sur l'axe de mesure (OC),
     - un boîtier (6) supportant le système optique de visée, la au moins une source lumineuse (2) et l'appareil de capture d'images (1),
     - l'axe optique de visée (OV) étant incliné d'un angle alpha par rapport à l'horizontale, l'angle alpha est compris entre +5 degrés et +85 degrés lorsque le boîtier (6) est posé sur une surface horizontale,
     - l'appareil de capture d'images et le calculateur étant adaptés pour mesurer des écarts pupillaires pour la première valeur de proximité P1 et pour la deuxième valeur de proximité P2 et au moins un paramètre de monturisation parmi la hauteur, l'angle pantoscopique, la distance verre-oeil et la position du centre de rotation de l'oeil.

2. Dispositif de mesure selon la revendication 1 comprenant des moyens de déplacement et/ou d'orientation de l'axe optique de visée (OV) de manière à aligner l'axe optique de mesure (OC) sur l'axe du regard du sujet.

3. Dispositif de mesure selon la revendication 1 ou la revendication 2 comprenant en outre des moyens de mesure de distance entre ledit dispositif et la tête du sujet, lesdits moyens de mesure de distance étant choisis parmi : un télémètre, un système de traitement d'image basé sur la qualité image, un système de traitement d'image basé sur la mesure de repères montés sur un clip (8) fixé à une monture (7), un système d'étalonnage ou un système de mesure de distance par ultrasons.

4. Dispositif de mesure selon l'une des revendications 1 à 3 dans lequel la au moins une source lumineuse (2) comprend au moins une source infrarouge (22) et en ce que l'appareil de capture d'images (1) est apte à capturer des images dans l'infrarouge.

5. Dispositif de mesure selon l'une des revendications 1 à 4 comprenant en outre un séparateur optique de faisceau (12, 23) disposé sur le trajet optique entre le sujet et la au moins une source (2), l'appareil de capture d'images (1), le séparateur optique de faisceau (12, 23) étant apte à combiner le faisceau d'éclairage et la première cible ou la deuxième cible sur l'axe optique de visée (OV) en direction des yeux du sujet, ledit séparateur de faisceau (12, 23) étant apte à diriger le faisceau de réfraction oculaire en direction de l'appareil de capture d'images (1) sur l'axe optique de mesure (OC).

6. Dispositif de mesure selon les revendications 4 et 5 dans lequel le séparateur optique de faisceau (12, 23) comporte un miroir dichroïque apte à transmettre le faisceau d'éclairage infrarouge et à réfléchir le faisceau cible.

7. Dispositif de mesure selon l'une des revendications 1 à 6 dans lequel l'angle entre l'axe optique de mesure (OC) et l'axe optique de visée (OV) est inférieur à 10 degrés.

**8.** Dispositif de mesure selon la revendication 7 dans lequel les axes optiques de mesure (OC) et de visée (OV) sont confondus.

**9.** Dispositif de mesure selon l'une des revendications 1 à 7 dans lequel l'appareil de capture d'images (1) est adapté pour former une image du visage du sujet sur un champ objet ayant un diamètre d'au moins 50 mm.

**10.** Procédé de mesure binoculaire d'au moins une caractéristique de réfraction oculaire objective d'un sujet pour une pluralité de distances de vision, ledit procédé comprenant les étapes suivantes :

- génération d'une première cible ayant une première valeur de proximité P1 suivant un axe optique de visée (OV) aligné avec l'axe du regard du sujet, l'axe du regard étant incliné d'un angle alpha1 compris entre +5 degrés et +85 degrés par rapport à l'horizontale ;
- génération d'un faisceau d'éclairage en direction des deux yeux du sujet,
- séparation et direction d'un faisceau de réfraction oculaire, formé par réfraction du au moins un faisceau d'éclairage sur les yeux du sujet, en direction de l'appareil de capture d'images (1) sur l'axe optique de mesure (OC),
- capture d'une première image de réfraction oculaire des deux yeux suivant une direction de mesure (OC) lorsque la première cible de proximité P1 est activée, le champ de ladite première image permettant la visualisation des deux yeux et d'au moins une partie du visage du porteur ;
- génération d'une deuxième cible ayant une deuxième valeur de proximité P2 suivant le même axe optique de visée (OV) destiné à être aligné avec l'axe du regard du sujet, l'axe du regard étant incliné d'un angle alpha2 compris entre +5 degrés et +85 degrés par rapport à l'horizontale, l'axe du regard restant centré sur l'axe de mesure (OC) et l'abaissement du regard variant par rapport à la tête du sujet ;
- capture d'au moins une deuxième image de réfraction oculaire des deux yeux suivant la même direction de mesure (OC) lorsque la deuxième cible de proximité P2 est activée, le champ de ladite deuxième image permettant la visualisation des deux yeux et d'au moins une partie du visage du porteur ;
- traitement numérique de la première image de réfraction oculaire et de la au moins une deuxième image de réfraction oculaire pour en déduire au moins une caractéristique de réfraction oculaire objective des deux yeux du sujet en fonction de la première valeur de proximité P1 et de la deuxième valeur de proximité P2, et en fonction d'un abaissement du regard par rapport à la tête du sujet, tout en maintenant l'axe du regard centré sur l'axe de mesure (OC),

le procédé comprenant en outre une étape de mesure des écarts pupillaires pour la première valeur de proximité P1 et pour la deuxième valeur de proximité P2 et/ou au moins un paramètre de monturisation parmi la hauteur, l'angle pantoscopique, la distance verre-oeil et la position du centre de rotation de l'oeil.

**11.** Procédé selon la revendication 10 dans lequel la capture d'une première image de réfraction oculaire est effectuée pour une première posture du sujet et dans lequel la capture d'une deuxième image de réfraction oculaire est effectuée pour une deuxième posture du sujet différente de la première posture et dans lequel les étapes de capture d'images sont réalisées dans des conditions où la tête du sujet est libre de contraintes physiques extérieures.

**12.** Procédé de mesure selon l'une des revendications 10 à 11 comprenant en outre une étape préliminaire de mise en place de manière à positionner et orienter le dispositif de mesure relativement au sujet.

**13.** Procédé de mesure selon l'une des revendications 10 à 12 comprenant une étape d'alerte en cas de défaut de positionnement relatif ou d'orientation relative entre le dispositif de mesure et le sujet.

**Patentansprüche**

**1.** Vorrichtung zum binokularen Messen mindestens einer Zielrefraktionseigenschaft des Auges eines Patienten für mehrere visuelle Bereiche, **dadurch gekennzeichnet, dass** die Vorrichtung umfasst:

- ein optisches Zielsystem (13, 23) von variabler Nähe, das geeignet ist, selektiv ein erstes Ziel mit einem ersten Wert einer Nähe P1 und mindestens ein zweites Ziel mit einem zweiten Wert einer Nähe P2 zu erzeugen, wobei das erste Ziel und das zweite Ziel auf einer einzigen und selben optischen Zielachse (OV) zentriert sind, das die Variation einer Senkung des Blicks des Patienten in Bezug zu seinem Kopf ermöglicht, wobei eine identische Blickachse beibehalten wird,
- mindestens eine Lichtquelle (2), die geeignet ist, mindestens einen Lichtstrahl in Richtung der zwei Augen des Patienten zu erzeugen,
- ein Bildaufnahmegerät (1), wobei das Gerät eine optische Messachse (OC) hat, die auf einer Blickachse des Patienten ausgerichtet ist, wobei das Bildaufnahmegerät angeordnet ist und ein Feld hat, das für die Aufnahme eines Bildes der zwei Augen und mindestens eines Teils des Gesichts des Trägers geeignet ist,

- wobei das Gerät geeignet ist, einen Refraktionsstrahl des Auges durch Refraktion des mindestens einen Lichtstrahls auf den Augen des Patienten zu empfangen, wobei das Bildaufnahmegerät geeignet ist, ein erstes okulares Refraktionsbild der zwei Augen aufzunehmen, wenn das erste Ziel der Nähe P1 aktiviert ist, und mindestens ein zweites okulares Refraktionsbild der zwei Augen aufzunehmen, wenn das zweite Ziel der Nähe P2 aktiviert ist,

- einen Rechner, der geeignet ist, das erste okulare Refraktionsbild und das zweite okulare Refraktionsbild zu empfangen, um davon eine Messung mindestens einer okularen Zielrefraktionseigenschaft der zwei Augen des Patienten in Abhängigkeit vom ersten Wert der Nähe P1 und vom zweiten Wert der Nähe P2 und in Abhängigkeit von einem Senken des Blicks des Patienten in Bezug zum Kopf, wobei die Blickachse auf die Messachse (OC) zentriert bleibt, abzuleiten,

- ein Gehäuse (6), das das optische Zielsystem, die mindestens eine Lichtquelle (2) und das Bildaufnahmegerät (1) trägt,

- wobei die optische Zielachse (OV) um einen Winkel Alpha in Bezug zur Horizontalen geneigt ist, wobei der Winkel Alpha zwischen +5 Grad und +85 Grad beträgt, wenn das Gehäuse (6) auf einer horizontalen Fläche steht,

- wobei das Bildaufnahmegerät und der Rechner geeignet sind, Pupillenabstände für den ersten Wert der Nähe P1 und für den zweiten Wert der Nähe P2 und mindestens einen Fassungsparameter unter der Höhe, dem Winkel, dem Glas-Aug-Abstand und der Position des Rotationszentrums des Auges zu messen.

2. Messvorrichtung nach Anspruch 1, umfassend Mittel zum Bewegen und/oder Ausrichten der optischen Zielachse (OV), um die optische Messachse (OC) auf der Blickachse des Patienten auszurichten.

3. Messvorrichtung nach Anspruch 1 oder Anspruch 2, ferner umfassend Mittel zum Messen des Abstands zwischen der Vorrichtung und dem Kopf des Patienten, wobei die Abstandsmessmittel ausgewählt sind unter: einem Telemeter, einem Bildbearbeitungssystem, basierend auf der Bildqualität, einem Bildbearbeitungssystem, basierend auf der Messung von Bezugseinheiten, die auf einem auf einer Fassung (7) montierten Clip (8) montiert sind, einem Eichungssystem oder einem Abstandsmesssystem mit Ultraschall.

4. Messvorrichtung nach einem der Ansprüche 1 bis 3, bei der mindestens eine Lichtquelle (2) mindestens eine Infrarotquelle (22) umfasst, wobei das Bildaufnahmegerät (1) geeignet ist, Bilder im Infrarotbereich aufzunehmen.

5. Messvorrichtung nach einem der Ansprüche 1 bis 4, ferner umfassend einen optischen Strahlenteiler (12, 23), der auf der optischen Strecke zwischen dem Patienten und mindestens einer Quelle (2) angeordnet ist, wobei das Bildaufnahmegerät (1), der optische Strahlenteiler (12, 23) geeignet ist, den Lichtstrahl und das erste Ziel oder das zweite Ziel auf der optischen Zielachse (OV) in Richtung der Augen des Patienten zu kombinieren, wobei der Strahlenteiler (12, 23) geeignet ist, den Refraktionsstrahl des Auges in Richtung des Bildaufnahmegeräts (1) auf der optischen Messachse (OC) zu lenken.

6. Messvorrichtung nach den Ansprüchen 4 und 5, bei der der optische Strahlenteiler (12, 23) einen dichroitischen Spiegel umfasst, der geeignet ist, den Infrarotlichtstrahl zu übertragen und den Zielstrahl zu reflektieren.

7. Messvorrichtung nach einem der Ansprüche 1 bis 6, bei der der Winkel zwischen der optischen Messachse (OC) und der optischen Zielachse (OV) kleiner als 10 Grad ist.

8. Messvorrichtung nach Anspruch 7, bei der die optischen Mess- (OC) und Zielachsen (OV) zusammenfallen.

9. Messvorrichtung nach einem der Ansprüche 1 bis 7, bei der das Bildaufnahmegerät (1) geeignet ist, ein Bild des Gesichts des Patienten auf einem Zielfeld mit einem Durchmesser von mindestens 50 mm zu bilden.

10. Verfahren zum binokularen Messen mindestens einer Zielrefraktionseigenschaft des Auges eines Patienten für mehrere visuelle Bereiche, wobei das Verfahren die folgenden Schritte umfasst:

- Erzeugung eines ersten Ziels mit einem ersten Wert einer Nähe P1 entlang einer optischen Zielachse (OV), die mit der Blickachse des Patienten ausgerichtet ist, wobei die Blickachse um einen Winkel alpha1 zwischen +5 Grad und +85 Grad in Bezug zur Horizontalen geneigt ist;

- Erzeugung eines Lichtstrahls in Richtung der zwei Augen des Patienten,

- Trennung und Ausrichtung eines okularen Refraktionsstrahls, der durch Refraktion des mindestens einen Lichtstrahls auf den Augen des Patienten gebildet ist, in Richtung des Bildaufnahmegeräts (1) auf der optischen Messachse (OC),

- Aufnahme eines ersten okularen Refraktionsbildes der zwei Augen in einer Messrichtung (OC), wenn das erste Ziel der Nähe P1 aktiviert ist, wobei das Feld des ersten Bildes die Sichtbarmachung der zwei Augen und mindestens eines Teils des Gesichts des Trägers ermöglicht;

- Erzeugung eines zweiten Ziels mit einem zweiten Wert einer Nähe P2 entlang derselben optischen Zielachse (OV), die dazu bestimmt ist, mit der Blickachse des Patienten ausgerichtet zu sein, wobei die Blickachse um einen Winkel alpha2 zwischen +5 Grad und +85 Grad in Bezug zur Horizontalen geneigt ist, wobei die Blickachse auf der Messachse (OC) zentriert bleibt, und das Senken des Blicks in Bezug zum Kopf des Patienten variiert;

- Aufnahme mindestens eines zweiten okularen Refraktionsbildes der zwei Augen entlang derselben Messrichtung (OC), wenn das zweite Ziel der Nähe P2 aktiviert ist, wobei das Feld des zweiten Bildes die Sichtbarmachung der zwei Augen und mindestens eines Teils des Gesichts des Trägers ermöglicht;

- digitale Bearbeitung des ersten okularen Refraktionsbildes und des mindestens einen zweiten okularen Refraktionsbildes, um davon mindestens eine okulare Zielrefraktionseigenschaft der zwei Augen des Patienten in Abhängigkeit vom ersten Wert der Nähe P1 und dem zweiten Wert der Nähe P2 und in Abhängigkeit von einem Senken des Blicks in Bezug zum Kopf des Patienten abzuleiten, wobei die Blickachse auf die Messachse (OC) zentriert bleibt,

wobei das Verfahren ferner einen Schritt der Messung der Pupillenabstände für den ersten Wert der Nähe P1 und für den zweiten Wert der Nähe P2 und/oder mindestens eines Fassungsparameters unter der Höhe, dem Winkel, dem Glas-Aug-Abstand und der Position der Rotationsmitte des Auges umfasst.

11. Verfahren nach Anspruch 10, bei dem die Aufnahme eines erste okularen Refraktionsbildes für eine erste Körperhaltung des Patienten erfolgt, und bei dem die Aufnahme eines zweiten okularen Refraktionsbildes für eine zweite Körperhaltung des Patienten, die zur ersten Körperhaltung unterschiedlich ist, erfolgt, und bei dem die Schritte der Bildaufnahme unter Bedingungen erfolgen, unter denen der Kopf des Patienten frei von äußeren physischen Spannungen ist.

12. Messverfahren nach einem der Ansprüche 10 bis 11, ferner umfassend einen vorherigen Anordnungsschritt, um die Messvorrichtung in Bezug zum Patienten zu positionieren und auszurichten.

13. Messverfahren nach einem der Ansprüche 10 bis 12, umfassend einen Alarmschritt im Falle einer relativen Fehlpositionierung oder einer relativen Fehlausrichtung zwischen der Messvorrichtung und dem Patienten.

## Claims

1. A binocular device for measuring at least one objective ocular refraction characteristic of a subject for a plurality of vision distances, **characterized in that** said device comprises:

- an optical system (13, 23) for generating a focus of variable proximity, able to generate selectively a first target having a first proximity value P1 and at least a second target having a second proximity value P2, said first target and said second target being centered on one and the same focus optical axis (OV), enabling variation of a lowering of gaze of the subject relative to his head, while maintaining a same gaze direction;
- at least one light source (2) able to generate at least one illuminating beam in the direction of the two eyes of the subject;
- an image-capturing apparatus (1), said apparatus having a measurement optical axis (OC) aligned with a gaze axis of the subject, the image-capturing apparatus being placed and having a field adapted for image acquisition of the two eyes and at least a portion of the face of the wearer
- said apparatus being suitable for receiving an ocular refraction beam by refraction of the at least one illuminating beam in the eyes of the subject, the image-capturing apparatus being suitable for capturing a first ocular refraction image of the two eyes when the first target of proximity P1 is activated and at least one second ocular refraction image of the two eyes when the second target of proximity P2 is activated; and
- a processor suitable for receiving the first ocular refraction image and the second ocular refraction image in order to deduce therefrom a measurement of at least one objective ocular refraction characteristic of the two eyes of the subject depending on the first proximity value P1 and the second proximity value P2, and as a function of lowering of gaze of the subject relative to his head, while maintaining the gaze direction centered on the measurement axis (OC)
- a case (6) holding the focus-generating optical system, the at least one light source (2) and the image-capturing apparatus (1),
- the focus optical axis (OV) being inclined at an angle alpha to the horizontal, the angle alpha being comprised between +5 degrees and +85 degrees when the case (6) is placed on a horizontal surface,
- the image-capturing apparatus and the processor being suitable for measuring pupillary distances for the first proximity value P1 and for the second proximity value P2 and at least one fitting parameter among height, pantoscopic angle, eye/glass distance and the position of the center of rotation of the eye.

2. The measuring device as claimed in claim 1, comprising means for moving and/or orienting the focus optical axis (OV) so as to align the measurement optical axis (OC) with the axis of gaze of the subject.

3. The measuring device as claimed in claim 1 or claim 2, furthermore comprising means for measuring the distance between said device and the head of the subject, said means for measuring distance being chosen from: a telemeter, an image-processing system based on image quality, an image-processing system based on the measurement of reference marks mounted on a clip (8) fastened to a frame (7), a gauging system or a system for measuring distance by ultrasound.

4. The measuring device as claimed in one of claims 1 to 3, in which the at least one light source (2) comprises at least one infrared source (22) and in which the image-capturing apparatus (1) is able to capture images in the infrared.

5. The measuring device as claimed in one of claims 1 to 4, furthermore comprising an optical beam splitter (12, 23) placed on the optical path between the subject and the at least one source (2), the image-capturing apparatus (1), the optical beam splitter (12, 23) being able to combine the illuminating beam and the first target or the second target on the focus optical axis (OV) in the direction of the eyes of the subject, said beam splitter (12, 23) being able to direct the ocular refraction beam toward the image-capturing apparatus (1) on the measurement optical axis (OC).

6. The measuring device as claimed in claims 4 and 5, in which the optical beam splitter (12, 23) comprises a dichroic mirror able to transmit the infrared illuminating beam and reflect the target beam.

7. The measuring device as claimed in one of claims 1 to 6, in which the angle between the measurement optical axis (OC) and the focus optical axis (OV) is smaller than 10 degrees.

8. The measuring device as claimed in claim 7, in which the measurement optical axis (OC) and the focus optical axis (OV) are merged.

9. The measuring device as claimed in one of claims 1 to 7, in which the image-capturing apparatus (1) is suitable for forming an image of the face of the subject on an object field having a diameter of at least 50 mm.

10. A binocular method for measuring at least one objective ocular refraction characteristic of a subject for a plurality of vision distances, said method comprising the following steps:

- generating a first target having a first proximity value P1 along a focus optical axis (OV) aligned with the axis of gaze of the subject, the axis of gaze being inclined at an angle alpha1 comprised between +5 degrees and +85 degrees to the horizontal;
- generating an illuminating beam in direction of the two eyes of the subject;- splitting and directing an ocular refraction beam formed by refraction of said at least one illuminating beam in the eyes of the subject, toward the image-capturing apparatus on the measurement optical axis.
- capturing a first ocular refraction image of the two eyes in a measurement direction (OC) when the first target of proximity P1 is activated, the field of said first image allowing visualization of the two eyes and at least a portion of the face of the wearer;
- generating a second target having a second proximity value P2 along the same focus optical axis (OV), said axis being intended to be aligned with the axis of gaze of the subject, the axis of gaze being inclined at an angle alpha2 comprised between +5 degrees and +85 degrees to the horizontal, the gaze direction remaining centered on the measurement axis (OC) and the lowering of gaze of the subject varying relative to his head ;
- capturing at least one second ocular refraction image of the two eyes in the same measurement direction (OC) when the second target of proximity P2 is activated, the field of said second image allowing visualization of the two eyes and at least a portion of the face of the wearer; and
- digitally processing the first ocular refraction image and the at least one second ocular refraction image in order to deduce therefrom at least one objective ocular refraction characteristic of the two eyes of the subject depending on the first proximity value P1 and the second proximity value P2, and as a function of lowering of gaze of the subject relative to his head, while maintaining the gaze direction centered on the measurement axis (OC),

the method furthermore comprising a step of measuring pupillary distances for the first proximity value P1 and for the second proximity value P2 and/or at least one fitting parameter among height, pantoscopic angle, eye/glass distance and the position of the center of rotation of the eye.

11. The method as claimed in claim 10, in which a first ocular refraction image is captured for a first posture of the subject and in which a second ocular refraction image is captured for a second posture of the subject different from the first posture, and in which the image-capturing steps are carried out under conditions where the head of the subject is free from external physical constraints.

12. The measuring method as claimed in one of claims 10 and 11, furthermore comprising a preliminary placing step so as to position and orient the measuring device relative to the subject.

13. The measuring method as claimed in one of claims 10 to 12, comprising an alerting step in case of relative mispositioning or relative misorientation of the measuring device and the subject.

Fig.1

# Fig.2a

# Fig.2b

**Fig.3**

**Fig.4**

## Fig.5

8  18  7  15  38  28

55mm

OD          OG

| Réfraction | | [dpt] |
|---|---|---|
| -2,25+1,50 82° | -1,25+1,50 98'' | |
| | | |
| Pupille | | [mm] |
| 4,1 | 4,6 | |
| Distance interpupillaire | | |
| | 55 | [mm] |

## Fig.6

OD  7  OG

33D  33G
32D  32G
31D  31G
30D  30G

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20030108350 A1
- EP 0305238 A1
- FR 2914173 A1
- FR 2952517 A1

**Littérature non-brevet citée dans la description**

- **KUSSEL J.** Light-intensity distribution in eccentric photorefraction crescents. *Opt. Soc. Am. A,* Juin 1998, vol. 15 (6